# EUROPEAN PATENT SPECIFICATION

(11) **EP 0 625 194 B1**
(45) Date of publication and mention of the grant of the patent: **24.04.1996**
(21) Application number: 93904711.4
(22) Date of filing: 04.02.1993
(51) Int. Cl.: C12N 15/11, C12N 9/00, A61K 31/70, C07H 21/00

(54) **ENHANCEMENT OF RIBOZYME CATALYTIC ACTIVITY BY A NEIGHBORING FACILITATOR OLIGONUCLEOTIDE**
Erhöhung der katalytischen Aktivität von Ribozymen mit einem benachbartem Oligonukleotid als Heffer
INTENSIFICATION DE L'ACTIVITE CATALYTIQUE DES RIBOZYMES EN UTILISANT UN OLIGONUCLEOTIDE DE FACILITATION VOISIN

(30) Priority: 04.02.1992 US 830713
(43) Date of publication of application: 23.11.1994
(73) Proprietor: THE WORCESTER FOUNDATION FOR BIOMEDICAL RESEARCH, Shrewsbury, MA 01545 (US)
(72) Inventor: GOODCHILD, John, Worcester, MA 01602 (US)
(74) Representative: Vossius, Volker, Dr.
(86) International application number: US9300783
(87) International publication number: WO9315194

(56) References cited:
- EP-A- 0 421 376
- FEBS LETTERS, vol. 238, no. 1, September 1988, Amsterdam (NL); I.V. KUTYAVIN et al., pp. 35-38
- NUCLEIC ACIDS RESEARCH, vol. 16, no. 8, 1988, Arlington, VA (US); L.J. MAHER III. et al., pp. 3341-3358
- ARCHIVES OF BIOCHEMISTRY & BIOPHYSICS, vol. 284, no. 2, 01 February 1991, New York, NY (US); J. GOODCHILD et al., pp. 386-391
- NUCLEIC ACIDS RESEARCH, vol. 18, no. 1, 11 January 1990, Arlington, VA (US); B.S. SPROAT et al., pp. 41-49
- NUCLEIC ACIDS RESEARCH, vol. 20, no. 17, 11 January 1992, Arlington, VA (US); J. GOODCHILD, pp. 4607-4612

## Description

This invention relates to ribozymes that cleave RNA, and more specifically to the enhancement of ribozyme catalytic activity using a facilitator oligonucleotide complementary to an RNA sequence near or contiguous to the RNA sequence to which the ribozyme hybridizes. Furthermore, the invention relates to compositions comprising a ribozyme and an effective amount of a facilitator oligonucleotide and to the use of a ribozyme and a facilitator oligonucleotide for the preparation of a pharmaceutical composition for treating humans and other mammals having various viral infections.

Drugs might be based on RNA catalysts (ribozymes) designed to cleave viral or messenger RNA with high specificity at a rapid rate. These requirements historically have been mutually limiting.

Ribozymes consist of a catalytic core having flanking sequences adjacent to the core which hybridize to the substrate RNA. The simplest catalytic core is an RNA motif known as a hammerhead.

Ribozyme specificity depends on the number of base pairs formed between the ribozyme flanking sequences and its RNA substrate. Increased base pairing has been shown to decrease the rate of cleavage. Goodchild and Kohli, Arch. Biochem. Biophys., 284: 386-391 (1991). Goodchild and Kohli studied the cleavage of a sequence from HIV-1 RNA by various hammerhead ribozymes and determined that the rate of cleavage was dependent on the length of the flanking sequence. Shorter sequences were shown to result in weaker binding between the ribozyme and the cleavage products together with increased rate of cleavage. A ribozyme with 12 base flanking sequences cleaved 10 times faster then one with 20 bases.

However, to have the requisite selectivity or specifity, i.e., the ability to discriminate between all RNA molecules in a cell, a ribozyme must form a minimum of about 15 base pairs with the target substrate. This requirement for selectivity limits the rate of cleavage that may be realized.

Accordingly, ribozymes having increased catalytic activity or methods of increasing ribozyme catalytic activity are needed.

Further, coordination of Mg⁺ or Mn⁺ to a ribozyme at the active site is normally required for the ribozyme to catalyze sequence specific phosphodiester cleavage of an external RNA oligonucleotide substrate. See Nature, 361: 182-185 (Lehman and Joyce, 1993). The required concentration of the Mg⁺ or Mn⁺ is about 20 mM. However, there is normally only about a 1-2 mM of these ions actually available in a cellular environment even though the cellular concentrations are usually in the range of 50-100 mM. Thus, the available concentrations of Mg⁺ and Mn⁺ are a factor that limits the catalytic efficiency of a ribozyme. Methods for overcoming the limiting availability of these ions and therefore enhancing ribozyme activity are essential.

Koizumi et al., FEBS Lett. 239: 285-288 (1988), discuss the design of two ribozymes for site-specific cleavage of RNA. A UA site in an undecaribonucleotide was cleaved by a ribozyme consisting of two partially paired oligoribonucleotides with chain lengths of 19 and 15. The other ribozyme, which consists of 19-mer and a 13-mer, recognized a UC sequence at positions 42 and 43 of 5 S rRNA.

Haseloff and Gerlach, Nature 334: 585-59 (1988), discuss the dissection of the RNA substrate and enzyme activities from a single self-cleaving domain from the (+) strand of the satellite RNA of tobacco ringspot virus (sTobRV). Inspection of the separated substrate and ribozyme activities, in comparison with other naturally-occurring self-cleaving domains, led to a model for the design of oligoribonucleotides which posses new and highly sequence-specific endoribonuclease activities. This model was successfully tested by the design and construction of ribozymes targeted against three sites within the Tn9 chloramphenicol acetyl-transferase (CAT) messenger RNA sequence.

Hampel and Tritz, Biochemistry 28: 4929-4933 (1989), identified an RNA catalytic domain within the sequence of the 359 base long negative-strand satellite RNA of tobacco ringspot virus. The catalytic domain contains two minimal sequences of RNA, a 50 base catalytic RNA sequence, and a 14 base substrate RNA sequence. The catalytic complex of catalytic RNA/substrate RNA represents a structure not previously found in any RNA catalytic reaction.

Hampel et al., Nucleic Acids Res. 18: 299-304 (1990) discuss the identification of the catalytic domain within the sequence of the negative strand of the satellite RNA of tobacco ringspot virus. Minimum energy RNA folding calculations predict a two dimensional model with four major helical regions which are supported by mutagenesis experiments. This model for the catalytic complex consists of a 50 base catalytic RNA and a 14 base substrate RNA folded together in a type of hairpin two dimensional structure. Part of the recognition region between the catalyst and substrate is two helices of 6 bases and 4 bases respectively. Catalytic activity remains when the bases in these two helices are changed but base pairing is maintained. Thus an appropriately engineered 'hairpin' catalyst is capable of cleaving heterologous RNA.

Uhlenbeck, Nature, 328: 596-600 (1987) describes the synthesis of two oligoribonucleotides that can combine to form a structure consistent with the consensus self-cleaving domain. Because rapid cleavage of one of the oligomers was observed only when the other was present, the domain was necessary and sufficient for cleavage. The properties of the cleavage reaction were studied in detail. Nearly complete cleavage occurred even with large excess of the oligomer that was cleaved. This indicates that the oligomer that is uncleaved can cycle in the reaction and therefore be considered to act as a catalyst in the cleavage of the other oligomer.

Fedor and Uhlenbeck, Proc. Natl. Acad. Sci. USA 87: 1668-1672 (1990), analyzed the kinetics of cleavage for several hammerhead sequences to characterize the reaction mechanism and explore how nucleotides involved in substrate binding affect cleavage.

Goodchild et al., Arch. Biochem. Biophys. 263: 401-409 (1988) discusses the effects of a series of synthetic oligonucleotides (hybridons) complementary to the 5' noncoding regions of rabbit β-globin mRNA on endogenous protein synthesis in a rabbit reticulocyte cell-free translation system. With highly purified hybridons inhibition was completely specific for beta globin. Mixtures of two oligonucleotides binding contiguously to the mRNA were more effective than either oligomer alone.

Maher and Dolnick, Nucleic Acids Res. 16: 3341-3358 (1988) report that antisense oligonucleotides containing either anionic diester or neutral methylphosphonate internucleoside linkages were prepared by automated synthesis, and subsequently compared for their ability to arrest translation of human dihydrofolate reductase (DHFR) mRNA in a nuclease treated rabbit reticulocyte lysate. In the case of oligodeoxyribonucleotides, tandem targeting of three 14-mers resulted in synergistic and complete selective inhibition of DHFR synthesis at a total oligomer concentration of 25 µM.

Kutyavin et al, FEBS Lett. 238: 35-38 (1988) report that mono- and diphenazinium derivatives of oligonucleotides complementary to the DNA sequence adjacent to the target sequence of the addressed alkylation of DNA significantly enhance the extent and specificity of alkylation by p-(N-2-chloroethyl-N-methylamino(benzylamido) derivatives of the addressing oligonucleotides.

The present invention provides methods for increasing ribozyme catalytic activity without reducing specificity, which methods comprise reacting an RNA molecule with a ribozyme and a facilitator oligonucleotide. In the inventive method, the facilitator oligonucleotide hybridizes with a sequence in the target RNA that is either contiguous to or near a sequence in the target RNA to which the ribozyme hybridizes.

The present invention further provides compositions comprising a ribozyme and an effective amount of a facilitator oligonucleotide.

The invention also includes compositions comprising a ribozyme and an effective amount of a facilitator oligonucleotide and the use of a ribozyme and a facilitator oligonucleotide for the preparation of a pharmaceutical composition for treating humans and other mammals having various viral infections.

The invention also provides methods for reducing the amount of magnesium (Mg⁺) or manganese (Mn⁺) that is required by the ribozyme for catalytic cleavage of target RNA.

Figure 1 shows the nucleotide sequences of substrate RNA (S₁), ribozyme (R) and facilitator oligodeoxynucleotides F₁, F₂, F₃, facilitator ribooligonucleotide F₄ and facilitator oligodeoxynucleotide F₅. The site of cleavage of substrate is indicated by the arrow.

Figure 2A is an autoradiograph showing the results of cleavage of radiolabelled substrate S₁ by ribozyme R without facilitator oligonucleotide to give products P₁ and P₂ containing 20 and 13 nucleotides respectively.

Figure 2B is an autoradiograph showing the results of cleavage of radiolabelled substrate S₁ by ribozyme R in the presence of facilitator oligonucleotide F₁ to give products P₁ and P₂ containing 20 and 13 nucleotides respectively.

Figure 3 is a graph showing the effect of chain length on the rate of cleavage using facilitator oligonucleotides F₁, F₂, and F₃ and a control reaction with no facilitator oligonucleotide.

Figure 4 is a graph comparing cleavage of substrate S₁ using a facilitator that hybridizes to a sequence in the target substrate S₁ that is contiguous to the S₁ sequence to which ribozyme R hybridizes (F₂) with cleavage by a facilitator that hybridizes to a sequence in S₁ that is spaced 3 nucleotides from the S₁ sequence to which ribozyme R hybridizes.

Figure 5 is a graph comparing the cleavage of S₁ by R using deoxyoligonucleotide (F₁) or ribooligonucleotide (F₄) facilitators with a control reaction with no facilitator.

Figure 6 is a graph comparing cleavage of S₁ by R using a facilitator having a phosphorothioate backbone with cleavage by a control with no facilitator.

Figure 7 shows the sequences of ribozymes R₄ and R₅ that differ only in the lengths of their flanking sequences (21 and 12 bases respectively).

Figure 8 is a graph comparing reactions catalyzed by R₄ and R₅. The extent of reaction is shown as the ratio of product P1 over R.

Figure 9a shows the sequences of ribozyme R₅ and facilitator F₁ hybridized to substrate S₁.

Figure 9b shows the sequence of a ribozyme R₁₈ prepared by covalently linking facilitator F₁ and ribozyme R₅.

Figure 10 is a graph comparing the rate of cleavage of substrate S₂ by R₅ alone, R₅ and F₁ together, and R₁₈ alone.

Figure 11 is a graph showing the effect of varying the Mg⁺ concentration and the effect of facilitator oligonucleotide on the rate of cleavage of substrate S₁ by ribozyme R₅.

The development of antiviral drugs based on RNA catalysts has been inhibited by the mutually limiting requirements of high specificity and RNA cleavage rate. Increased base pairing between a ribozyme and a substrate RNA has been shown to decrease the rate of RNA cleavage. In order for a ribozyme to discriminate between all RNAs in a cell, a ribozyme must form about 15 base pairs with the target. However, longer flanking sequences in ribozymes is related to decreased catalytic cleavage.

It has been unexpectedly discovered that the rate of cleavage of substrate RNA by a ribozyme is enhanced by introducing an oligonucleotide into the system which hybridizes either near or immediately adjacent to the target RNA sequence to which the ribozyme hybridizes. By facilitor oligonucleotide is meant an oligonucleotide that hybridizes with a sequence in the target RNA that is either contiguous to or near a sequence in the target RNA to which the ribozyme hybridizes. Thus, the facilitator oligonucleotide binds to a sequence in the target RNA that is spaced no more than about five nucleotides from the target RNA sequence to which the ribozyme hybridizes.

It has also been unexpectedly discovered that the use of a facilitator oligonucleotide with a ribozyme reduces the amount of magnesium ion (Mg⁺) that is required by the ribozyme to catalytically cleave target RNA sequences. The rate of cleavage of target RNA by a ribozyme is dramatically enhanced when target RNA is reacted with the ribozyme in the presence of a facilitator oligonucleotide.

The facilitator oligonucleotides suitable for use in the instant invention may be either deoxyoligonucleotides or ribo-oligonucleotides. Furthermore, the facilitator oligonucleotide may be selected to bind to a sequence contiguous to the flanking sequence either at the 5' or the 3' side of the ribozyme. In addition, a combination of two facilitator oligonucleotides may be employed, where one facilitator is bound contiguously to the 3' flanking sequence and the other to the 5' flanking sequence. Alternatively, a plurality of facilitators may be employed to catalyze ribozyme activity. For example, in a system employing three facilitators, two facilitators could binc contiguously to the 3' flanking sequence (a second facilitator would hybridize contiguous to a first facilitator hybridized adjacent the 3' flanking sequence of the ribozyme), while a third facilitator could bind contiguously to the 5' flanking sequence. A variety of other combinations are possible.

The facilitator oligonucleotides of the present invention typically comprise from about 5 to 50 nucleotides. More preferred facilitator oligonucleotides comprise from about 5 to 15 nucleotides. Particularly preferred facilitators according to the invention comprise about 13 nucleotides. Selection of a facilitator of a specific length is related to the length of the ribozyme flanking sequences.

Preferred facilitator oligonucleotides containing phosphorothioate linkages comprise from about 15 to 30 nucleotides. Particularly preferred oligonucleotides containing phosphorothioate linkages comprise from about 20 to 25 nucleotides.

The facilitator deoxynucleotides may be selected to have from about 5 to 50 nucleotides complementary to the RNA substrate sequence as well as additional nucleotides which are not complementary to the RNA sequence. The additional nucleotides not complementary to the RNA sequence do not hybridize with the target RNA.

The ribozymes of the invention are normally those that cleave related target RNA sequences. The specific facilitator oligonucleotides may be synthesized to hybridize with the desired RNA sequences such that the facilitator hybridizes with a sequence in the target RNA that is contiguous to a sequence in the target RNA to which the ribozyme hybridizes. As described here, the oligonucleotides can be synthesized on automated DNA synthesizers or from DNA templates.

In addition, the facilitator oligonucleotides may be synthesized such that they do not hybridize to a sequence that is contiguous to the flanking sequence of the desired ribozyme. For example, the facilitator may be synthesized such that, when the ribozyme and facilitator oligonucleotide are bound to the substrate RNA, a small gap of from one to about five oligonucleotides exists between the ribozyme and the facilitator oligonucleotide. In other words, the facilitator oligonucleotide hybridizes with a sequence in the target RNA that is spaced about one to five oligonucleotides from the target RNA sequence to which the ribozyme hybridizes.

The facilitator oligonucleotides may be synthesized and subsequently modified to include moieties which will influence the rate of substrate cleavage by ribozyme, increase uptake by cells, or increase resistance to degradation. The facilitator oligonucleotides may be prepared to contain substituted nucleotide analogs or modified nucleic acid backbones, resulting in chimeric oligonucleotides. The modifications may be either natural or synthetic. The oligonucleotides may optionally be chemically capped at either the 3' end or 5' end to yield a chemically capped oligonucleotide.

The facilitator oligonucleotides may be modified to contain a nucleotide having a substituent on the oxygen at the 2'-position of the nucleotide; i.e., by introducing a 2'-O-substituted nucleotide into the facilitator. The substituent on the nucleotide 2'-oxygen may be a lower alkyl group, lower alkenyl group, a phenyl alkyl group where the alkyl is lower alkyl, a phenyl alkenyl group where the alkenyl is lower alkenyl, an acyl group, or a phenylacyl group. Suitable synthetic methods for preparing various 2'-O-substituted nucleotides are disclosed by Iribarren et al., Proc. Natl. Acad. Sci. 87: 7747-7751 (1990); Sproat et al., Nucleic Acids Res. 19: 733-738 (1991); and Sproat et al., Nucleic Acids Res. 18: 41-49 (1989). Inclusion of 2'-O-alkylnucleotides stabilizes hybrids as well as increases resistance to degradation by nucleases.

By increasing the number of bases of the substrate RNA hybridized near the cleavage site, facilitators permit use of faster acting ribozymes with shorter flanking sequences. Facilitators might be of dual benefit in also directing cleavage of the target RNA by endogenous ribonuclease H. While the method of the invention has been demonstrated using sequences from HIV-1 RNA, it is applicable to sequences from other sources. Thus, facilitator sequences may be chosen to enhance the catalytic rate of ribozymes that cleave target RNA that code for a variety of proteins that lead to disease.

This technology is applicable to any disease situation where antisense nucleotides may be used. I.e., the combination of facilitators and ribozyme may be employed in any disease situation where it is desired to inhibit the synthesis of a protein.

In the methods of the invention, the facilitator oligonucleotides apparently do not dissociate from the target RNA and therefore do not appear to catalytically recycle. This apparent lack of dissociation thus requires employment of at least about a stoichiometric amount of facilitator oligonucleotide. The ribozyme, however, does recycle and thus allows for use of only a catalytic amount of ribozyme in the invention.

The present invention also includes compositions which comprise a ribozyme and an effective amount of a facilitator oligonucleotide and the use thereof for the preparation of such pharmaceutical compositions. The invention further provides methods for treating mammals infected with a pathogenic organism such as a virus comprising administering to the mammal a ribozyme and a facilitator oligonucleotide in amounts effective to cleave target RNA, where the facilitator oligonucleotide hybridizes with a sequence in the target RNA that spaced no more than about five nucleotides from the target RNA to which the ribozyme hybridizes. For optimum activity, the facilitator should be used in an amount that is approximately stoichiometric with the amount of target RNA sequence while the ribozyme may be used in only a catalytic amount, e.g., the ribozyme concentration may be about 5% of the facilitator concentration.

In any treatment the compositions comprising the ribozyme and facilitator oligonucleotide must be administered to the mammal or individuals in a manner capable of delivering the oligonucleotide and ribozyme initially into the blood stream and subsequently into cells. Methods for preparing and administering compositions according to the invention that will be effective in vivo are easily determined.

Antisense oligonucleotides very similar to facilitators and ribozymes are taken up by cells and can block the expression of target RNA. See e.g., J. Goodchild in Oligonucleotides, Antisense Inhibitors of Gene Expression, pp 53-77, J. Cohen, editor, 1989, Macmillan Press, London; and Toulme in Antisense RNA and DNA, pp. 175-194, J.A.H. Murray, editor, 1992, Wiley-Liss, New York. It has been demonstrated that antisense oligonucleotides can block the expression of target RNA's in whole animals. J. Clin. Invest. 88: 1190-1196 (Burch and Mahan 1991). See Antisense Research and Development 1: 349-350 (Whitesell et al., 1991); and Science 258: 1792-1795 (Kitajima et al., 1992). It is also well known that ribozymes are able to cleave a target RNA in vitro, including HIV-1 RNA, and also in cells with resulting inhibition of viral replication or of expression of the target RNA. See, J. Virol. 66: 1432-144 (Dropulic et al., 1992); Nuc. Acids Res. 20: 4559-4565 (Taylor et al., 1992); Proc. Nat. Acad. Sci. USA 89: 10802-10806 (Ojwang et al., 1992); Gene, 117: 179-184 (Koizume et al., 1992); Journal of Virology, 65: 5531-5534 (Weerasinghe et al., 1991); Proc. Nat. Acad. Sci. USA, 86: 9139-9143 (Cameron and Jennings, 1989); EMBO Journal, 12: 3861-3866 (Cotton and Birnstiel 1989); and Science, 247: 1222-1225 (Saver et al., 1990).

The compositions of the invention may be administered parenterally, orally, by inhalation or spray or rectally in dosage unit formulations containing conventional non-toxic pharmaceutically acceptable carriers, adjuvants and vehicles. The term "parenteral" as used herein includes subcutaneous injections, intravenous, intramuscular, intrasternal injection or infusion techniques. The compositions of the invention would be provided in a pharmaceutical formulation comprising the composition and a pharmaceutically acceptable carrier. In order for the compositions to be suitable for oral administration, oligonucleotides and ribozymes must be resistant to nucleases. Such resistance to nucleases may be imparted to the oligonucleotides and ribozymes by, for example, internucleotide phosphate modifications. Modified internucleotide phosphates suitable for use in the facilitator oligonucleotides of the present invention include phosphorothioates, alkylphosphorothioates such as for example, methylphosphorothioates, alkylphosphonates such as, for example, methylphosphonates, phosphoramidates, and phosphotriesters.

The amount of active composition that may be combined with the carrier materials to produce a single dosage form will vary depending upon the host treated and the particular mode of administration. It will be understood that the specific dose level for any particular patient will depend upon a variety of factors including the activity of the specific composition employed, the age, body weight, general health, sex, diet, time of administration, route of administration, severity of the particular disease undergoing therapy. The compositions of the invention comprising ribozyme and facilitator oligonucleotides can be effectively administered intravenously or subcutaneously using doses of from about 0.1 mg/kg to about 50 mg/kg to yield facilitator concentrations in the patient of from about 0.01 µM to about 100 µM.

One skilled in the art will recognize that modifications may be made in the present invention without deviating from the spirit or scope of the invention. The invention is illustrated further by the following examples which are not to be construed as limiting the invention or scope of the specific procedures described herein.

### Exemple 1

### 1. Preparation of RNA Substrate

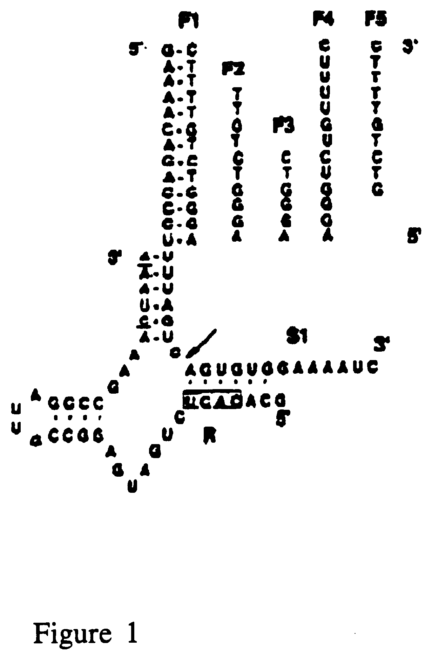

A synthetic RNA substrate strand (S₁) was prepared to correspond to the sequence 146-173 in HIV-1 RNA; the sequence of which is shown in Figure 1. This RNA substrate strand was transcribed from synthetic DNA templates following a method described by Milligan and Uhlenbeck, Nucleic Acids Res. 15: 8783-8798 (1987), in a reaction containing Tris·HCl (40 mM, pH 8.1) , MgCl₂ (6 mM), spermidine (1 mM), dithiothreitol (50 mM), bovine serum albumin (50 µg per ml), inorganic pyrophosphatase (4 units per ml), T7 RNA polymerase (4000 units per ml) and four ribonucleotide 5'-triphosphates (1 µM each) supplemented with α-³P-UTP (3000 Ci/mmol). After incubation at 37°C for 2 hours, the RNA was purified by electrophoresis in 10% polyacrylamide gels containing 8 M urea. The radiolabeled RNA was quantitated using the specific activity of the incorporated ³P.

### 2. Preparation of Hammerhead Ribozyme

A hammerhead ribozyme (R) designed to cleave RNA substrate strand S₁ was prepared. The sequence of ribozyme R is shown in Figure 1. The hammerhead ribozyme was prepared by automated chemical synthesis using standard phosphoramidite reagents. Products were purified by electrophoresis in 15% polyacrylamide gels containing 8M urea, eluted by crush and soak in 0.5M ammonium acetate, desalted and quantitated by UV absorption.

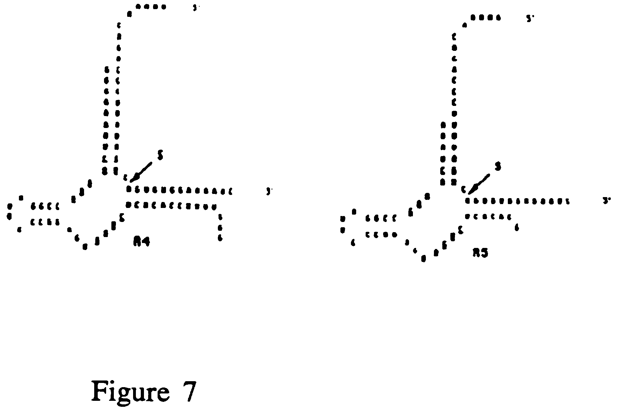

Hammerhead. ribozymes R₄ and R₅, the sequence of each of which is shown in Figure 7, were designed to cleave a substrate strand and prepared using essentially the procedure described above for ribozyme R.

### 3. Preparation of Facilitator and Control Oligonucleotides

Facilitator oligonucleotides F₁, F₂, and F₃ were prepared to contain 13, 10, and 6 nucleotides respectively, and to hybridize to substrate S contiguously with ribozyme R. Facilitator oligoribonucleotide F₄ was prepared with the same sequence as F₁. In addition, a control oligonucleotide having a random sequence was synthesized. The sequences of oligonucleotides F₁, F₂, F₃, and F₄ are shown in Figure 1.

Both ribo- and deoxyoligonucleotides were prepared by automated chemical synthesis utilizing essentially the same procedures set forth in part 2 of this Example.

Facilitator oligonucleotide F₅ was prepared using essentially the same procedure as facilitators F₁-F₄ and contained 10 nucleotides. The sequence of F₅ is shown in Figure 1.

### Example 2

### Preparation of Ribozvmes R₁₈ and R₅

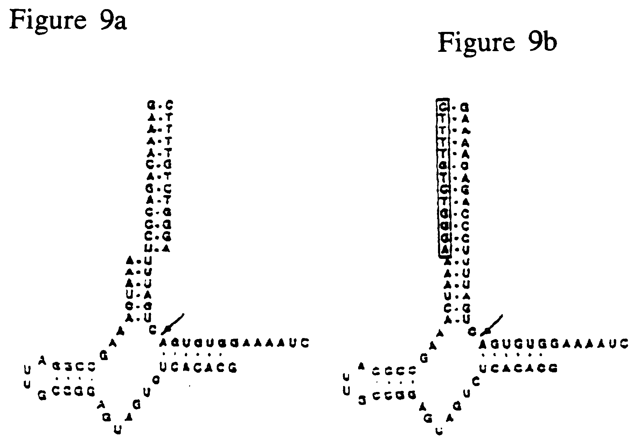

- A ribozyme consisting of a 35 nucleotide sequence (R₅) was prepared essentially according to the procedure set forth in part 2 of Example 1 above. A 48 nucleotide ribozyme, R₁₈, was prepared by covalently coupling R₅ with facilitator F₁ (prepared above). The sequences of ribozymes R₅, R₁₈ and facilitator, F₁, are shown in Figures 9a and 9b.

### Example 3

### Cleavage of Substrate RNA

The cleavage of substrate RNA by ribozyme R was studied both with and without facilitator oligo F₁. The cleavage of substrate RNA gave products P₁ and P₂ having chain lengths expected from cleavage at the site indicated in Figure 1.

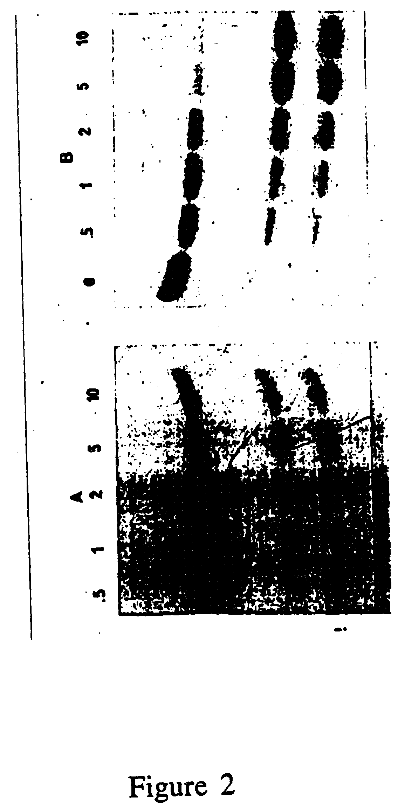

The cleavage reactions were run as follows: a solution (45 µl) containing substrate (13.4 µM), ribozyme (0.67 µM) and facilitator where appropriate (20 µM) in 50 mM Tris·HCl (pH 7.4) was brought to 37°C. Reaction was initiated by the addition of MgCl₂ (5 µl 200 mM). After times of 0.5, 1, 2, 5, and 10 minutes, aliquots of 5 µl were added to 15 µl of saturated urea:200 mM EDTA (1:1) and cooled to about -70°C with dry ice to stop the reaction. The samples were then denatured by heating in formamide loading buffer at 90°C for 3 minutes and subsequently analyzed alongside molecular weight markers by electrophoresis in 15% polyacrylamide gel containing 7M urea. The products were autoradiographed. The autoradiographs are shown in Figure 2. Panel A shows the results of the cleavage reaction without any facilitator oligonucleotide and Panel B shows the results of cleavage with facilitator oligonucleotide F₁.

### Example 4

### Relation of Facilitator Length to Ribozyme Activity

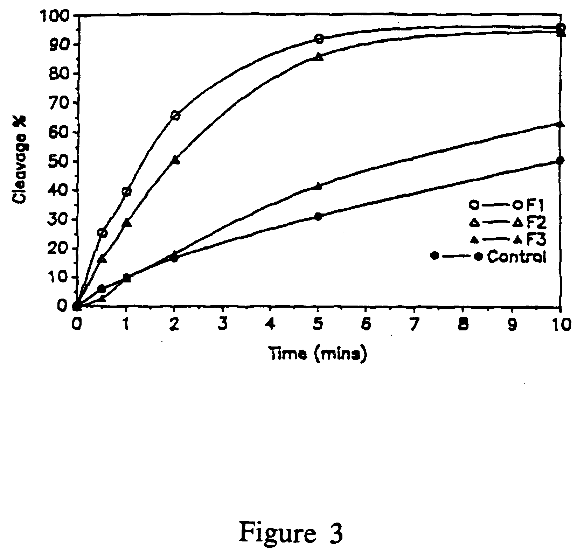

Cleavage of substrate RNA by ribozyme R was determined in the presence of facilitator oligonucleotides (F₁, F₂, F₃, and F₄) of varying length. Cleavage reactions were run under conditions substantially similar to those employed in Example 2 above. Products and starting materials were quantitated for each time point. Autoradiograph gels were sliced and the materials on the slices quantitated by scintillation counting. The results of this experiment are graphically shown in Figure 3.

Cleavage with no facilitator reached about 94% completion after about 160 minutes. The facilitator of 13 deoxynucleotides significantly reduced reaction half life. Table 1 shows the time required for ribozyme to cleave 10 equivalents of substrate at 37°C. The longest facilitator, F₁, reduced this half life time from 10 minutes to 1.3 minutes. The effects of facilitators F₁-F₃ were inversely related to their lengths. A control oligonucleotide of the same length as F₁ had no effect on the rate.

In a separate experiment, Example 7, it was found that oligodeoxynucleotide F₁ was more effective at catalyzing ribozyme activity than oligoribonucleotide F₄ having the same sequence.

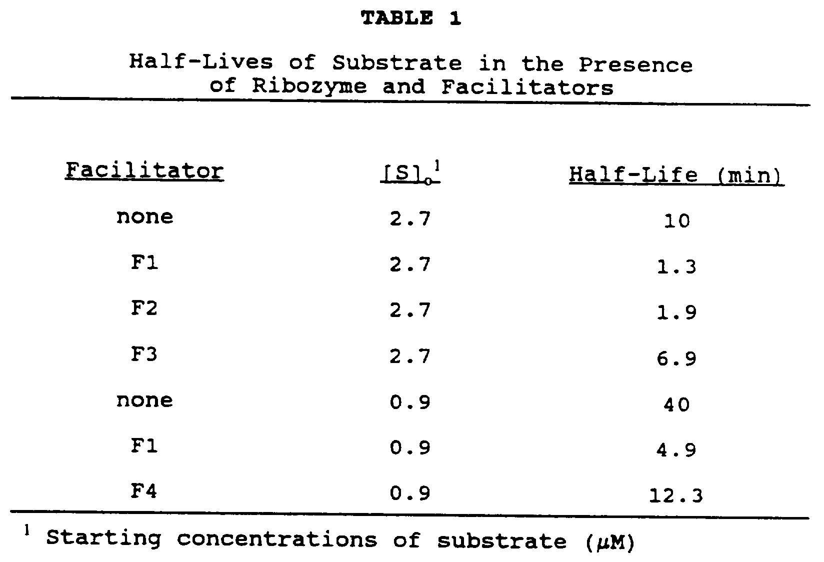

### Example 5

### Relation of Ribozyme Length and Activity and Facilitator to Cleavage Rate

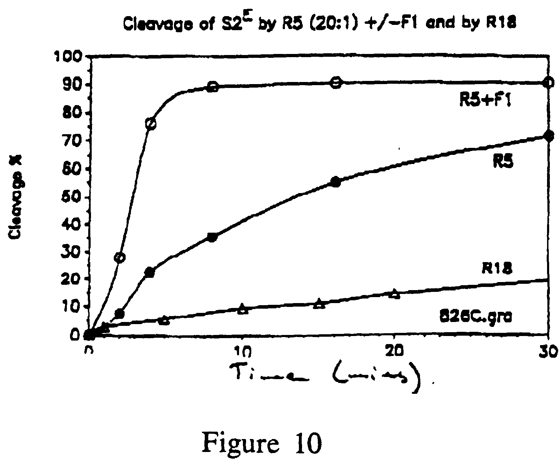

The rate of cleavage of target RNA (S) by ribozymes R₅ and R₁₈ was compared. Cleavage reactions were run as described in Example 3. The following three reactions were performed:
1. Cleavage with Ribozyme R₅ and facilitator F₁.
2. Cleavage with Ribozyme R₅ alone.
3. Cleavage with Ribozyme R₁₈ alone.
The results are shown in Figure 10. The ribozyme and separate facilitator cleaved target RNA at a much faster rate than the single ribozyme although the total number of base pairs formed with the target was the same in both cases.

### Example 6

### Cleavage by Ribozyme and Facilitator spaced from ribozyme

The cleavage of substrate RNA(S₁₎ was studied using ribozyme (R) and a facilitator F₅ that hybridizes to a sequence in S₁ that is spaced 3 nucleotides from the S₁ sequence to which R hybridizes.

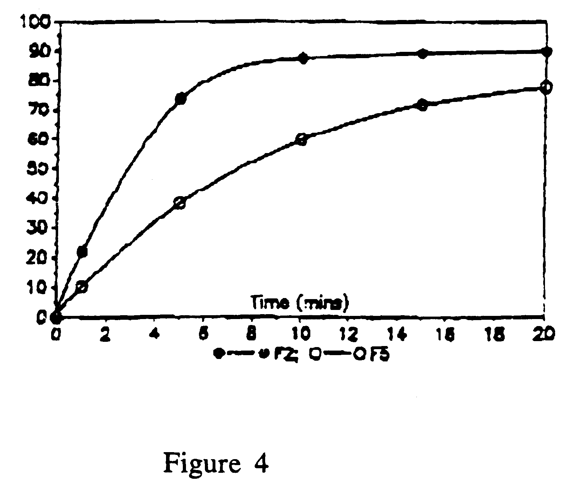

The cleavage reactions were run essentially as described above in Example 3. Each reaction contained 20 equivalents of S₁ and 30 of facilitator relative to R. A control reaction contained no facilitator. The initial concentration of S was 2.7 µM and reactions were performed in 50 mM Tris, pH 7.4 and 20 mM MgCl₂ at 37°C. The results are shown in Figure 4.

### Example 7

### Cleavage of Substrate by Ribozyme in the Presence of Facilitator Comprising Ribooligonucleotides and Deoxyoligonucleotides

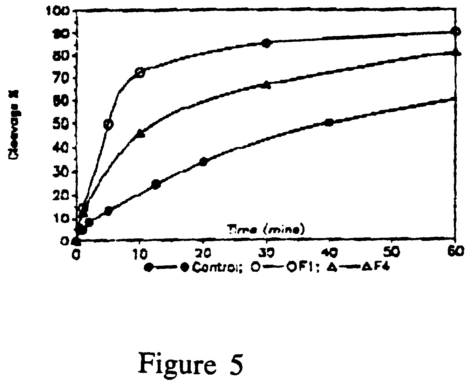

The rates of cleavage in the presence of deoxyoligonucleotide (F₁) and ribooligonucleotide (F₄) facilitator, and a control with no facilitator were studied. Reactions were performed essentially as described above in Example 6 using an initial substrate S₁ concentration of 1.6 µM. The results are shown in Figure 5.

### Example 8

### Cleavage by Ribozyme in the presence of facilitator having a phosphorothioate backbone

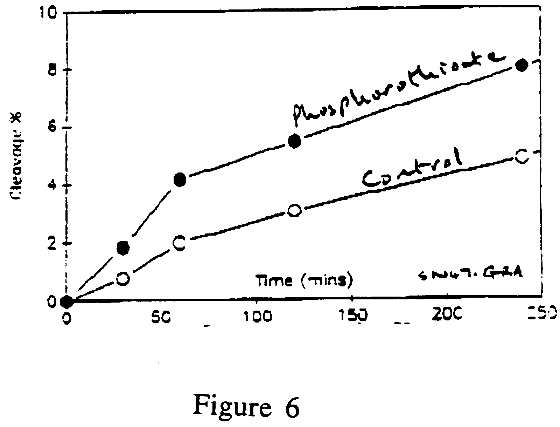

Cleavage of substrate RNA was studied using a facilitator having a phosphorothioate linkage as part of its backbone and compared to a control reaction having no facilitator. The reactions were run essentially as described in Example 6 at a temperature of 22°C. The results are shown in Figure 6.

### Example 9

### Relation between Ribozyme length and Activity

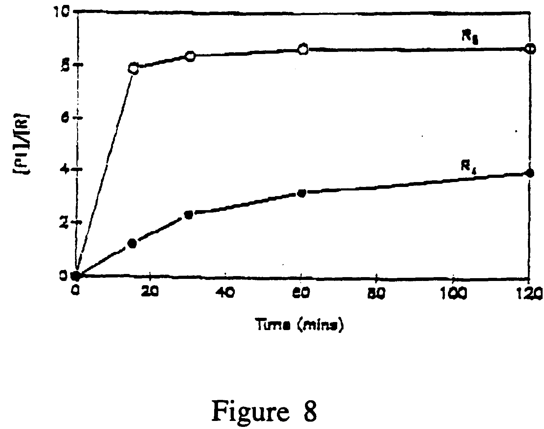

The rate of cleavage by ribozyme R₄ (21 nucleotides) was compared to that of ribozyme R₅ (12 nucleotides) using reaction conditions essentially as set forth in Example 6, above. The initial concentrations were: 19 nM ribozyme and 190 nM substrate. Results are shown in Figure 8 as the ratio of the concentration of product, P₁ over R.

### Example 10

### Relation of Facilitator to [mg⁺] Required for Cleavage

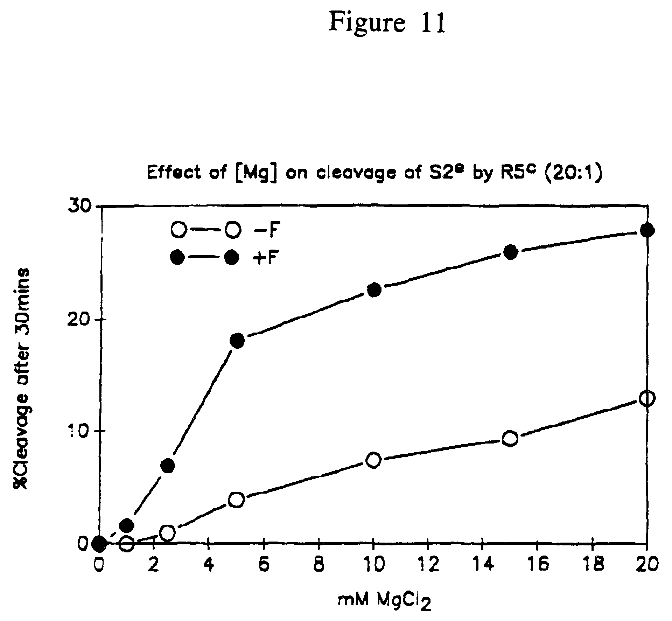

The rate of substrate (S₁) cleavage by ribozyme R₅ was studied with and without facilitator at various concentrations of Mg⁺. The reactions were run essentially as described above in Example 6, but at different concentrations of Mg⁺. The results are shown in Figure 11.

From the foregoing, it will appreciated that although specific embodiments of the invention have been described herein for purposes of illustration, various modifications may be made without deviating from the spirit or scope of the invention.

## Claims

1. A method for increasing the catalytic activity of a ribozyme comprising reacting a target RNA molecule with the ribozyme and a facilitor oligonucleotide, where the facilitor oligonucleotide hybridizes with a sequence in the target RNA that is spaced no more than about five nucleotides from the target RNA sequence to which the ribozyme hybridizes.

2. A method according to Claim 1, wherein the facilitator oligonucleotide comprises from about 5 to about 50 nucleotides.

3. A method according to Claim 2, wherein the facilitator oligonucleotide comprises from about 5 to about 15 nucleotides.

4. A composition comprising a ribozyme and an effective amount of a facilitator oligonucleotide.

5. A composition according to Claim 4, wherein the facilitator oligonucleotide hybridizes with a sequence in the target RNA that is spaced no more than about five nucleotides from the target RNA sequence to which the ribozyme hybridizes.

6. A composition according to Claim 5, wherein the facilitator oligonucleotide comprises from about 5 to about 50 nucleotides.

7. A composition according to Claim 6, wherein the facilitator oligonucleotide comprises from about 5 to about 15 nucleotides.

8. A facilitator oligonucleotide having the structure AGGGTC that binds to a sequence in a target RNA that is contiguous to a sequence in the target RNA to which the ribozyme hybridizes.

9. A facilitator oligonucleotide having the structure AGGGTCTGTT that binds to a sequence in a target RNA that is contiguous to a sequence in the target RNA to which the ribozyme hybridizes.

10. A facilitator oligonucleotide having the structure AGGTCTGTTTTC that binds to a sequence in a target RNA that is contiguous to a sequence in the target RNA to which the ribozyme hybridizes.

11. A method according to Claim 1, wherein the facilitator oligonucleotide comprises a modified internucleotide phosphate.

12. A method according to Claim 11, wherein the modified internucleotide phosphate is selected from the group consisting of phosphorothioates, alkylphosphorothioates, alkylphosphonates, phosphoramidates, and phosphotriesters.

13. A composition according to Claim 4, wherein the facilitator oligonucleotide comprises a modified internucleotide phosphate.

14. A composition according to Claim 13, wherein the modified internucleotide phosphate comprises a phosphorothioate, methylphosphonate, phosphoramidate, or phosphotriester.

15. A method for reducing the concentration of Mg⁺ and Mn⁺ required by a ribozyme for catalytic cleavage of target RNA comprising treating the target RNA with the ribozyme and a facilitator oligonucleotide that hybridizes with a sequence in the target RNA that is spaced no more than about five nucleotides from the target RNA sequence to which the ribozyme hybridizes.

16. A method for cleaving target RNA with a ribozyme comprising reducing the concentration of an ion selected from the group consisting of Mg⁺ and Mn⁺ by treating the target RNA with the ribozyme in the presence of a facilitator oligonucleotide that hybridizes with a sequence in the target RNA that is spaced no more than about five nucleotides from the target RNA sequence to which the ribozyme hybridizes.

17. A method for inhibiting the replication of target retroviral RNA comprising reacting the target RNA with a ribozyme and a facilitator oligonucleotide that hybridizes with a sequence in the target RNA that is spaced no more than about five nucleotides from the target RNA sequence to which the ribozyme hybridizes.

18. The use of a ribozyme and a facilitator oligonucleotide in an amount effective to cleave target RNA, where the facilitator oligonucleotide hybridizes with a sequence in the target RNA that is spaced no more than about five nucleotides from the target RNA sequence to which the ribozyme hybridizes for the preparation of a pharmaceutical composition for combatting infections caused by pathogenic organisms.

19. The use according to claim 18 wherein the facilitator oligonucleotide is used in an amount that is stoichiometric with the target RNA.

## Patentansprüche

1. Verfahren zur Erhöhung der katalytischen Aktivität eines Ribozyms, umfassend das Umsetzen eines Target-RNA-Moleküls mit dem Ribozym und einem Helfer-Oligonucleotid, wobei das Helfer-Oligonucleotid mit einer Sequenz in der Target-RNA hybridisiert, die nicht mehr als etwa 5 Nucleotide von der Target-RNA-Sequenz entfernt ist, mit der das Ribozym hybridisiert.

2. Verfahren nach Anspruch 1, worin das Helfer-Oligonucleotid von etwa 5 bis etwa 50 Nucleotide umfaßt.

3. Verfahren nach Anspruch 2, worin das Helfer-Oligonucleotid von etwa 5 bis etwa 15 Nucleotide umfaßt.

4. Zusammensetzung umfassend ein Ribozym und eine wirksame Menge eines Helfer-Oligonucleotids.

5. Zusammensetzung nach Anspruch 4, worin das Helfer-Oligonucleotid mit einer Sequenz in der Target-RNA hybridisiert, die nicht mehr als etwa 5 Nucleotide von der Target-RNA-Sequenz entfernt ist, mit der das Ribozym hybridisiert.

6. Zusammensetzung nach Anspruch 5, worin das Helfer-Oligonucleotid von etwa 5 bis etwa 50 Nucleotide umfaßt.

7. Zusammensetzung nach Anspruch 6, worin das Helfer-Oligonucleotid von etwa 5 bis etwa 15 Nucleotide umfaßt.

8. Helfer-Oligonucleotid mit der Struktur AGGGTC, das an eine Sequenz in einer Target-RNA bindet, die an eine Sequenz in der Target-RNA angrenzt, mit der das Ribozym hybridisiert.

9. Helfer-Oligonucleotid mit der Struktur AGGGTCTGTT, das an eine Sequenz in einer Target-RNA bindet, die an eine Sequenz in der Target-RNA angrenzt, mit der das Ribozym hybridisiert.

10. Helfer-Oligonucleotid mit der Struktur AGGTCTGTTTTC, das an eine Sequenz in einer Target-RNA bindet, die an eine Sequenz in der Target-RNA angrenzt, mit der das Ribozym hybridisiert.

11. Verfahren nach Anspruch 1, worin das Helfer-Oligonucleotid ein modifiziertes Internucleotidphosphat umfaßt.

12. Verfahren nach Anspruch 11, worin das modifizierte Internucleotidphosphat ausgewählt ist aus der Gruppe bestehend aus Phosphorothioaten, Alkylphosphorothioaten, Alkylphosphonaten, Phosphoramidaten und Phosphortriestern.

13. Zusammensetzung nach Anspruch 4, worin das Helfer-Oligonucleotid ein modifiziertes Internucleotidphosphat umfaßt.

14. Zusammensetzung nach Anspruch 13, worin das modifizierte Internucleotidphosphat ein Phosphorothioat, Methylphosphonat, Phosphoramidat oder einen Phosphortriester umfaßt.

15. Verfahren zur Reduzierung der Konzentration an Mg⁺ und Mn⁺, die von einem Ribozym zur katalytischen Spaltung von Target-RNA benötigt wird, umfassend das Behandeln der Target-RNA mit dem Ribozym und einem Helfer-Oligonucleotid, das mit einer Sequenz in der Target-RNA hybridisiert, die nicht mehr als etwa 5 Nucleotide von der Target-RNA-Sequenz entfernt ist, mit der das Ribozym hybridisiert.

16. Verfahren zur Spaltung von Target-RNA mit einem Ribozym, umfassend das Reduzieren der Konzentration eines Ions ausgewählt aus der Gruppe bestehend aus Mg⁺ und Mn⁺, durch Behandeln der Target-RNA mit dem Ribozym in Anwesenheit eines Helfer-Oligonucleotids, das mit einer Sequenz in der Target-RNA hybridisiert, die nicht mehr als etwa 5 Nucleotide von der Target-RNA-Sequenz entfernt ist, mit der das Ribozym hybridisiert.

17. Verfahren zur Inhibition der Replikation retroviraler Target-RNA, umfassend das Umsetzen der Target-RNA mit einem Ribozym und einem Helfer-Oligonucleotid, das mit einer Sequenz in der Target-RNA hybridisiert, die nicht mehr als etwa 5 Nucleotide von der Target-RNA-Sequenz entfernt ist, mit der das Ribozym hybridisiert.

18. Verwendung eines Ribozyms und eines Helfer-Oligonucleotids in einer Menge, die zur Spaltung von Target-RNA wirksam ist, wobei das Helfer-Oligonucleotid mit einer Sequenz in der Target-RNA hybridisiert, die nicht mehr als etwa 5 Nucleotide von der Target-RNA-Sequenz entfernt ist, mit der das Ribozym hybridisiert, zur Herstellung einer pharmazeutischen Zusammensetzung zur Bekämpfung von Infektionen, die durch pathogene Organismen hervorgerufen werden.

19. Verwendung nach Anspruch 18, worin das Helfer-Oligonucleotid in einer Menge verwendet wird, die zu der Target-RNA in einem stöchiometrischen Verhältnis steht.

## Revendications

1. Procédé pour l'intensification de l'activité catalytique d'un ribozyme, comprenant la réaction d'une molécule d'ARN cible avec le ribozyme et d'un oligonucléotide de facilitation, procédé dans lequel l'oligonucléotide de facilitation s'hybride avec une séquence dans l'ARN cible qui n'est pas éloignée de plus d'environ cinq nucléotides de la séquence d'ARN cible avec laquelle le ribozyme s'hybride.

2. Procédé selon la revendication 1, dans lequel l'oligonucléotide de facilitation comprend d'environ 5 à environ 50 nucléotides.

3. Procédé selon la revendication 2, dans lequel l'oligonucléotide de facilitation comprend d'environ 5 à environ 15 nucléotides.

4. Composition comprenant un ribozyme et une quantité efficace d'un oligonucléotide de facilitation.

5. Composition selon la revendication 4, dans laquelle l'oligonucléotide de facilitation s'hybride avec une séquence dans l'ARN cible qui n'est pas éloignée de plus d'environ cinq nucléotides de la séquence d'ARN cible avec laquelle le ribozyme s'hybride.

6. Composition selon la revendication 5, dans laquelle l'oligonucléotide de facilitation comprend d'environ 5 à environ 50 nucléotides.

7. Composition selon la revendication 6, dans laquelle l'oligonucléotide de facilitation comprend d'environ 5 à environ 15 nucléotides.

8. Oligonucléotide de facilitation ayant la structure AGGGTC qui se fixe à une séquence dans un ARN cible qui est contiguë à une séquence dans l'ARN cible à laquelle le ribozyme s'hybride.

9. Oligonucléotide de facilitation ayant la structure AGGGTCTGTT qui se fixe à une séquence dans un ARN cible qui est contiguë à une séquence dans l'ARN cible à laquelle le ribozyme s'hybride.

10. Oligonucléotide de facilitation ayant la structure AGGTCTGTTTTC qui se fixe à une séquence dans un ARN cible qui est contiguë à une séquence dans l'ARN cible à laquelle le ribozyme s'hybride.

11. Procédé selon la revendication 1, dans lequel l'oligonucléotide de facilitation comprend un phosphate internucléotidique modifié.

12. Procédé selon la revendication 11, dans lequel le phosphate internucléotidique modifié est choisi dans le groupe consistant en phosphorothioates, alkylphosphorothioates, alkylphosphonates, phosphoroamidates et phosphotriesters.

13. Composition selon la revendication 4, dans laquelle l'oligonucléotide de facilitation comprend un phosphate internucléotidique modifié.

14. Composition selon la revendication 13, dans laquelle le phosphate internucléotidique modifié comprend un phosphorothioate, méthylphosphonate, phosphoramidate ou phosphotriester.

15. Procédé pour la réduction de la concentration en Mg⁺ et Mn⁺ requise par un ribozyme pour la coupure catalytique d'ARN cible, comprenant le traitement de l'ARN cible avec le ribozyme et un oligonucléotide de facilitation qui s'hybride avec une séquence dans l'ARN cible qui n'est pas espacée de plus d'environ cinq nucléotides de la séquence d'ARN cible avec laquelle le ribozyme s'hybride.

16. Procédé pour la coupure d'ARN cible avec un ribozyme, comprenant la réduction de la concentration d'un ion choisi dans le groupe consistant en Mg⁺ et Mn⁺ par traitement de l'ARN cible avec le ribozyme en présence d'un oligonucléotide de facilitation qui s'hybride avec une séquence de l'ARN cible qui n'est pas espacée de plus d'environ cinq nucléotides de la séquence d'ARN cible avec laquelle le ribozyme s'hybride.

17. Procédé pour l'inhibition de la réplication d'ARN rétroviral cible, comprenant la réaction de l'ARN cible avec un ribozyme et un oligonucléotide de facilitation qui s'hybride avec une séquence de l'ARN cible qui n'est pas espacée de plus d'environ cinq nucléotides de la séquence d'ARN cible avec laquelle le ribozyme s'hybride.

18. Utilisation d'un ribozyme et d'un oligonucléotide de facilitation en une quantité efficace pour la coupure d'ARN cible, dans laquelle l'oligonucléotide de facilitation s'hybride avec une séquence dans l'ARN cible qui n'est pas espacée de plus d'environ cinq nucléotides de la séquence d'ARN cible avec laquelle le ribozyme s'hybride, pour la préparation d'une composition pharmaceutique pour lutter contre les infections provoquées par des organismes pathogènes.

19. Utilisation selon la revendication 18, dans laquelle l'oligonucléotide de facilitation est utilisé en une quantité qui est stoechiométrique avec l'ARN cible.
